# EUROPEAN PATENT SPECIFICATION

(11) **EP 3 728 261 B1**
(45) Date of publication and mention of the grant of the patent: **06.10.2021**
(21) Application number: 18829264.3
(22) Date of filing: 13.12.2018
(51) Int. Cl.: C07D 487/04, A61K 31/437, A61P 35/00

(54) **CO-CRYSTAL OF AN ORALLY AVAILABLE JANUS KINASE INHIBITOR**
ZWITTERKRISTALL EINES ORAL VERFÜGBAREN JANUS KINASE INHIBITOREN
CO-CRISTAL D'INHIBITEUR DE JANUS KINASE DISPONIBLE PAR VOIE ORALE

(30) Priority: 20.12.2017 EP 17208942; 15.05.2018 EP 18172429; 10.10.2018 EP 18199511
(43) Date of publication of application: 28.10.2020
(73) Proprietor: Sandoz AG, 4056 Basel (CH)
(72) Inventor: ENKELMANN, Dennis, Dimo, 6020 Innsbruck (AT)
(74) Representative: Prüfer & Partner mbB Patentanwälte · Rechtsanwälte
(86) International application number: PCT/EP2018/084691
(87) International publication number: WO 2019/121290

(56) References cited:
- EP-A1- 3 269 719
- WO-A1-2009/114512
- CN-A- 105 601 635

## Description

### FIELD OF THE INVENTION

The present invention relates to a co-crystal of baricitinib with lactic acid and to a process for its preparation. Furthermore, the invention relates to a pharmaceutical composition comprising said baricitinib lactic acid co-crystal and at least one pharmaceutically acceptable excipient. The pharmaceutical composition of the present invention can be used as a medicament, in particular for the treatment and/or prevention of inflammatory conditions such as e.g. rheumatoid arthritis (RA), atopic dermatitis (eczema) and systemic lupus erythematosus (SLE).

### BACKGROUND OF THE INVENTION

Baricitinib is an orally available Janus kinase (JAK) inhibitor. The Janus kinase family members (JAK1, JAK2, JAK3 and TYK2) are activated after stimulation of several cellular receptors by their specific growth factors, growth hormones, chemokines and cytokines. After activation, they phosphorylate STAT transcription factors and thereby substantially contribute to the immunologic processes in inflammatory diseases. Furthermore, JAK inhibitors have been found to mediate significant hair regrowth in patients suffering from alopecia areata.

Baricitinib is chemically also designated 2-[1-(Ethylsulfonyl)-3-[4-(7H-pyrrolo[2,3-d]pyrimidin-4-yl)-1H-pyrazol-1-yl]azetidin-3-yl]acetonitrile and can be represented by the chemical structure as depicted in formula A:

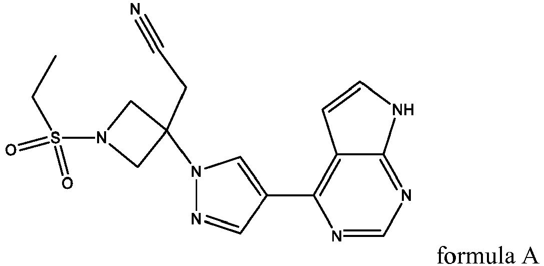

WO 2009/114512 A1 discloses processes for the preparation of baricitinib free base, which is obtained as an off-white solid in unknown form in Examples 70 and 78. In addition, Example 71 of the same application provides a process for the preparation of baricitinib phosphate, which is obtained as a white crystalline solid having a melting point of 187 °C. Finally, a process for the preparation of baricitinib trifluoroacetate is given in Example 1 of WO 2009/114512 A1 which is obtained in unknown state.

CN105601635 discloses crystalline Form A, Form H and Form I of baricitinib phosphate and methods for their preparation. According to the application, experimental repetition of Example 71 of WO 2009/114512 resulted in a crystalline form of baricitinib phosphate, which is denominated "Form X" in CN105601635, a PXRD of said "Form X" being displayed in Figure 16 of CN105601635.

In CN105566332 two crystalline forms of baricitinib trifluoroacetate designated Form A and Form B as well as methods for their preparation are disclosed. According to said application repetition of Example 1 of WO 2009/114512 A1 leads to a "semi-crystal" of baricitinib trifluoroacetate, a PXRD of said "semi-crystal" being displayed in Figure 15 of CN CN105566332.

Finally, solid forms of the free base are disclosed in applications WO 2015/145286 A1, WO 2015/166434 A1, WO 2016/141891, CN105693731 and IP.com article IPCOM000244270D.

Different solid-state forms of an active pharmaceutical ingredient (API) often possess different physical and chemical properties such as but not limited to dissolution rate, solubility, chemical stability, physical stability, hygroscopicity, melting point, morphology, flowability, bulk density and compressibility. Apart from conventional solid-state forms of an API, such as polymorphs, pseudopolymorphs (hydrates and solvates) and salts, pharmaceutical co-crystals open up further opportunities for customizing the physicochemical properties of APIs with a process or clinical need. For example, they can be tailored to enhance drug product bioavailability and stability and to enhance the processability of APIs during drug product manufacture.

Co-crystals are structurally readily distinguishable from salts because unlike salts, their components are in a neutral state and interact nonionically.Instead, co-crystals are structurally more similar to solvates and hydrates, in that both contain more than one component in the crystal lattice and the interaction between these components is nonionic. From a physical chemistry perspective, co-crystals can be viewed as a special case of solvates and hydrates, wherein the second component, the co-crystal former, is nonvolatile, (see also "Regulatory Classification of Pharmaceutical Co-Crystals", Guidance for Industry, FDA, Revision 1, August 2016).

Baricitinib (brand name Olumiant®) was approved by the EMA in February 2017. According to the European public assessment report (EPAR) Olumiant® capsules contain baricitinib in form of its crystalline free base (see EMA/13493/2017). It is further stated in the EPAR that baricitinib is only slightly soluble in 0.1 N HCl and practically insoluble in water.

WO 2015/166434 A1, WO 2016/141891 A1, CN105693731 A, and IPCOM000244270D disclose a crystalline form of baricitinib free base, therein designated as "form A". Said form suffers from certain drawbacks e.g. as reported in IPCOM000244270D, the water content varies from about 0 to 3% depending on conditions of its formation and isolation.

It is thus an objective of the present invention to provide an improved solid-state form of baricitinib, in particular a solid-state form of baricitinb with improved dissolution and solubility properties. A further objective of the present invention concerns the provision of an improved solid-state form of baricitinib which is anhydrous and non-hygroscopic irrespective of its previous preparation and isolation. It is a further objective of the present invention to provide an improved solid-state form of baricitinib which is physically and chemically stable, essentially free from organic solventsand/or characterized by improved powder characteristics such as flowability, bulk density and compressibility.

### SUMMARY OF THE INVENTION

The invention solves one or more of the above defined objectives by providing a pharmaceutically acceptable co-crystal of baricitinib with lactic acid. The co-crystal of the present invention possesses one or more improved physicochemical properties selected from dissolution rate, solubility, chemical stability, physical stability, hygroscopicity, melting point, morphology, flowability, bulk density and compressibility. In particular, the lactic acid co-crystal of the current invention shows a superior disolution rate in aqueous medium compared to crystalline baricitinib free base.

### Abbreviations

- PXRD: powder X-ray diffractogram
- SXRD: single X-ray diffraction
- FTIR: Fourier transform infrared
- ATR: attenuated total reflection
- DSC: differential scanning calorimetry
- TGA: thermogravimetric analysis
- NMR: nuclear magnetic resonance
- RT: room temperature
- RH: relative humidity
- API: active pharmaceutical ingredient
- THF: tetrahydrofuran

### Definitions

As used herein the term "room temperature" refers to a temperature in the range of from 20 to 30 °C.

The term "baricitinib" as used herein refers to 2-[1-(Ethylsulfonyl)-3-[4-(7H-pyrrolo[2,3-d]pyrimidin-4-yl)-1H-pyrazol-1-yl]azetidin-3-yl]acetonitrile according to the chemical structure depicted in formula A disclosed herein above.

The term "co-crystal" as used herein refers to crystalline materials composed of two or more different molecular and/or ionic compounds in the same crystal lattice that are associated by nonionic and noncovalent bonds, wherein at least two of the individual molecular and/or ionic compounds are solids at room temperature.

The terms "baricitinib co-crystal with lactic acid" or "co-crystal of baricitinib with lactic acid" or "baricitinib lactic acid co-crystal" as used interchangeably herein refer to a crystalline compound comprising baricitinib as active pharmaceutical ingredient and *D*- or *L*-lactic acid, as co-crystal former in the same crystal lattice, wherein the interaction between baricitinib and lactic acid is of nonionic and noncovalent nature.

The term "baricitinib form A" as used herein, refers to the crystalline form of baricitinib, which is disclosed in WO 2015/166434 A1, WO 2016/141891 A1, CN105693731 A, and IPCOM000244270D. Form A of baricitinib can be characterized by having a powder X-ray diffractogram comprising reflections at 2-Theta angles of (16.70 ± 0.2)°, (19.09 ± 0.2)°, (25.31 ± 0.2)° and (25.57 ± 0.2)°, when measured at a temperature in the range of from 20 to 30 °C with Cu-Kalpha_{1,2} radiation having a wavelength of 0.15419 nm.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-50% relative humidity.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 7.2° 2-Theta for example can appear between 7.0° and 7.4° 2-Theta, preferably between 7.1 and 7.3° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "essentially the same" with reference to Fourier transform infrared spectroscopy means that variabilities in peak positions and relative intensities of the peaks are to be taken into account. For example, a typical precision of the wavenumber values is in the range of ± 2 cm⁻¹. Thus, a peak at 3482 cm⁻¹ for example can appear in the range of from 3484 to 3480 cm⁻¹ on most infrared spectrometers under standard conditions. Differences in relative intensities are typically smaller compared to X-ray diffraction. However, one skilled in the art will appreciate that small differences in peak intensities due to degree of crystallinity, sample preparation and other factors can also occur in infrared spectroscopy. Relative peak intensities should therefore be taken as qualitative measure only.

The term "essentially the same" with reference to Raman spectroscopy means that variabilities in peak positions and relative intensities of the peaks are to be taken into account. For example, a typical precision of the wavenumber values is in the range of ± 3 cm⁻¹. Thus, a peak at 1595 cm⁻¹ for example can appear in the range of from 1592 to 1598 cm⁻¹ on most Raman spectrometers under standard conditions. Differences in relative intensities are typically smaller compared to X-ray diffraction. However, one skilled in the art will appreciate that small differences in peak intensities due to degree of crystallinity, sample preparation and other factors can also occur in Raman spectroscopy. Relative peak intensities should therefore be taken as qualitative measure only.

The term "solid-state form" as used herein refers to any crystalline and/or amorphous phase of a compound. Crystalline phases include anhydrous/non-solvated forms of a compound and their polymorphs, hydrates and solvates of a compound and their polymorphs, salts of a compound and their polymorphs, hydrates and solvates,and co-crystals of a compound and their polymorphs, hydrates and solvates, and any mixtures thereof.

As used herein, the term "essentially free of any other solid-state form" with reference to the composition comprising the baricitinib lactic acid co-crystal of the present invention, means that the baricitinib lactic acid co-crystal contains at most 20 weight%, preferably at most 10 weight%, more preferably at most 5 weight%, 4 weight%, 3 weight%, 2 weight% or 1 weight% of any other solid-state form of baricitinib, in particular baricitinib form A, based on the weight of the composition.

The terms "anhydrous" or "anhydrate" as used herein refer to a crystalline solid where no water is cooperated in or accommodated by the crystal structure. Anhydrous forms may still contain residual water, which is not part of the crystal structure but may be adsorbed on the surface or absorbed in disordered regions of the crystal. Typically, an anhydrous form does not contain more than 1.0 weight%, preferably not more than 0.5 weight% and most preferably not more than 0.3 weight%, 0.2 weight% or 0.1 weight% of water, based on the weight of the crystalline form. The water content can be determined by Karl-Fischer Coulometry.

The term "non-solvated" as used herein, when talking about a crystalline solid indicates that no organic solvent is cooperated in or accommodated by the crystal structure. Non-solvated forms may still contain residual organic solvents, which are not part of the crystal structure but may be adsorbed on the surface or absorbed in disordered regions of the crystal. Typically, a non-solvated form does not contain more than 1.0 weight%, preferably not more than 0.5 weight%, and most preferably not more than 0.3 weight%, 0.2 weight% or 0.1 weight% of organic solvents, based on the weight of the crystalline form. The organic solvent content can be determined ¹H-NMR.

The baricitinib lactic acid co-crystal may be referred to herein as being characterized by a powder X-ray diffractogram, a Fourier transform infrared spectrum and/or a Raman spectrum "as shown in" a figure. The person skilled in the art understands that factors such as variations in instrument type, response and variations in sample directionality, sample concentration, sample purity, sample history and sample preparation may lead to variations, for example relating to the exact reflection or peak positions and intensities. However, a comparison of the graphical data in the figures herein with the graphical data generated for an unknown physical form and the confirmation that two sets of graphical data relate to the same crystal form is well within the knowledge of a person skilled in the art.

As used herein, the term "mother liquor" refers to the solution remaining after crystallization of a solid from said solution.

A "predetermined amount" as used herein with regard to baricitinib lactic acid co-crystal of the present invention refers to the initial amount of the baricitinib lactic acid co-crystal used for the preparation of a pharmaceutical composition having a desired dosage strength of baricitinib.

As used herein, the term "effective amount" in conjunction with the baricitinib lactic acid co-crystal of the present invention encompasses an amount of the baricitinib lactic acid co-crystal which causes the desired therapeutic or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient. Excipients may take the function of vehicle, diluent, release agent, disintegrating agent, dissolution modifying agent, absorption enhancer, stabilizer or a manufacturing aid among others. Excipients may include fillers (diluents), binders, disintegrants, lubricants and glidants.

The terms "filler" or "diluent" as used herein refer to substances that are used to dilute the active pharmaceutical ingredient prior to delivery. Diluents and fillers can also serve as stabilizers.

As used herein the term "binder" refers to substances which bind the active pharmaceutical ingredient and pharmaceutically acceptable excipient together to maintain cohesive and discrete portions.

The terms "disintegrant" or "disintegrating agent" as used herein refers to substances which, upon addition to a solid pharmaceutical composition, facilitate its break-up or disintegration after administration and permits the release of the active pharmaceutical ingredient as efficiently as possible to allow for its rapid dissolution.

The term "lubricant" as used herein refers to substances which are added to a powder blend to prevent the compacted powder mass from sticking to the equipment during tableting or encapsulation process. They aid the ejection of the tablet from the dies and can improve powder flow.

The term "glidant" as used herein refers to substances which are used for tablet and capsule formulations in order to improve flow properties during tablet compression and to produce an anti-caking effect.

### BRIEF DESCRIPTION OF THE FIGURES

**Figure 1**: illustrates a representative PXRD of the baricitinib lactic acid co-crystal according to the present invention. The x-axis shows the scattering angle in °2-Theta, the y-axis shows the intensity of the scattered X-ray beam in counts of detected photons.
**Figure 2****:** illustrates a representative FTIR spectrum of the baricitinib lactic acid co-crystal according to the present invention. The x-axis shows the wavenumbers in cm⁻¹, the y-axis shows the relative intensity in percent transmittance.
**Figure 3****:** illustrates a representative Raman spectrum of the baricitinib lactic acid co-crystal according to the present invention. The x-axis shows the wavenumbers in cm⁻¹, the y-axis shows the Raman intensity.

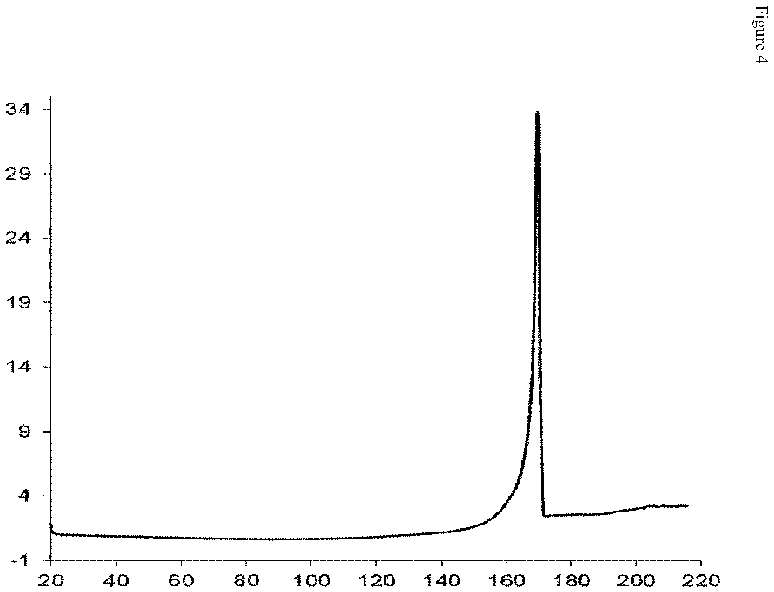

**Figure 4****:** illustrates a representative DSC curve of the baricitinib lactic acid co-crystal according to the present invention obtained from Example 1. The x-axis shows the temperature in degree Celsius (°C), the y-axis shows the heat flow rate in Watt per gram (W/g) with endothermic peaks going up.

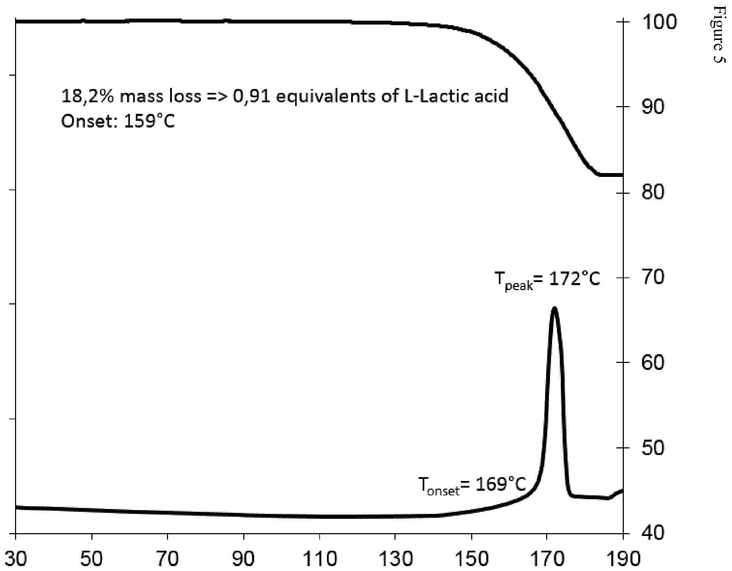

**Figure 5****:** illustrates a representative DSC curve and a TGA curve of the baricitinib lactic acid co-crystal according to the present invention obtained from Example 9 integrated in a single plot. The x-axis shows the temperature in degree Celsius (°C), the y-axis shows the heat flow rate in Watt per gram (W/g; left scale) with endothermic peaks going up and mass loss in percent (%; right scale), respectively.
**Figure 6****:** illustrates the calibration curve for the photometric measurement of the dissolution behavior. The y-axis shows light absorption at 310 nm, the x-axis shows the concentration of baricitinib in mmol/1.
**Figure 7**: illustrates the different dissolution behaviour of of the baricitinib lactic acid co-crystal and pure crystalline baricitinib free base Form A within the first 20 minutes. The y-axis shows the concentration of baricitinib mmol/1 determined by light absorption at 310 nm, the x-axis shows the time after mixing of the buffer with the test compounds in minutes.

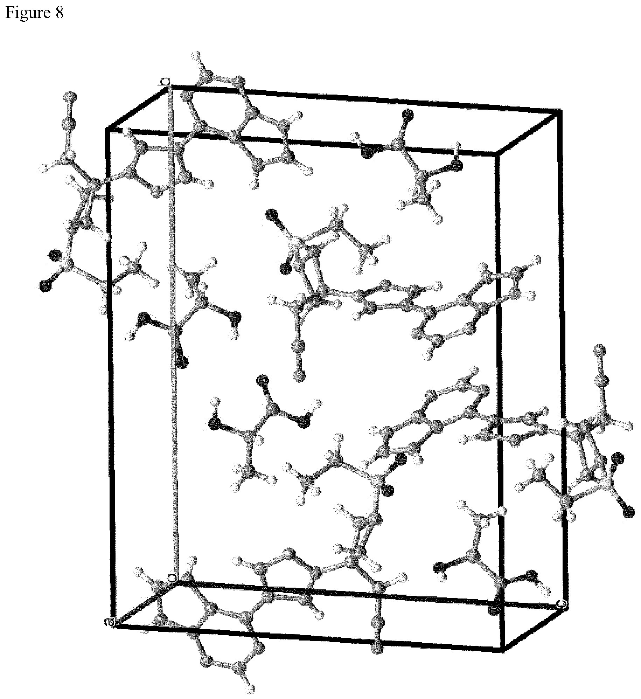

**Figure 8****:** illustrates the unit cell of the baricitinib lactic acid co-crystal of the present invention.

### DETAILED DESCRIPTION OF THE INVENTION

The present invention provides a pharmaceuticaly acceptable co-crystal composed of baricitinib as the active pharmaceutical ingredient and lactic acid, preferentially *L*-lactic acid as co-crystal former.

The baricitinib lactic acid co-crystal of the present invention shows an excellent dissolution kinetic in physiologically relevant aqueous media. In addition, said co-crystal is of anhydrous nature and non-hygroscopic and therefore has properties making the co-crystal of the invention useful for pharmaceutical formulation. Furthermore, the baricitinib lactic acid co-crystal of the present invention shows no thermal events in a DSC experiment until it starts to melt at about 168°C, indicating thermal stability. Finally, the baricitinib lactic acid co-crystal of the present invention demonstrates good flowability, high bulk density and good compressibility. All in all, these favorable attributes allow for a robust formulation and ensure a reliable safety and efficacy profile of a drug product containing the baricitinib lactic acid co-crystal of the present invention during the whole shelf-life of the product.

The baricitinib lactic acid co-crystal of the present invention may be characterized by analytical methods well known in the field of the pharmaceutical industry for characterizing crystalline solids. Such methods comprise but are not limited to powder and single X-ray diffraction, Fourier transform infrared spectroscopy, Raman spectroscopy, and DSC. The co-crystal of the present invention may be characterized by one of the aforementioned analytical methods or by combining two or more of them. In particular, the co-crystal of the present invention may be characterized by any one of the following embodiments or by combining two or more of the following embodiments.

In a first aspect, the invention relates to a co-crystal of baricitinib with lactic acid, in particular wherein the molar ratio of baricitinib to lactic acid is about 1:1. The lactic acid may be selected from *L*-lactic acid, D-lactic acid or mixtures thereof. Preferably the lactic acid is *L*-lactic acid.

In one embodiment, the invention relates to a co-crystal of baricitinib with *L*-lactic acid characterized by having the chemical structure as depicted in formula B-1 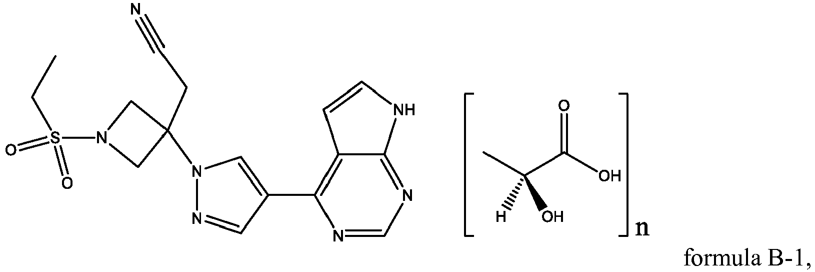 wherein n is in the range of from 0.8 to 1.2, preferably of from 0.9 to 1.1, even more preferably of from 0.95 to 1.05 and most preferably n is 1.0.

In another embodiment, the invention relates to a co-crystal of baricitinib with *D*-lactic acid characterized by having the chemical structure as depicted in formula B-2 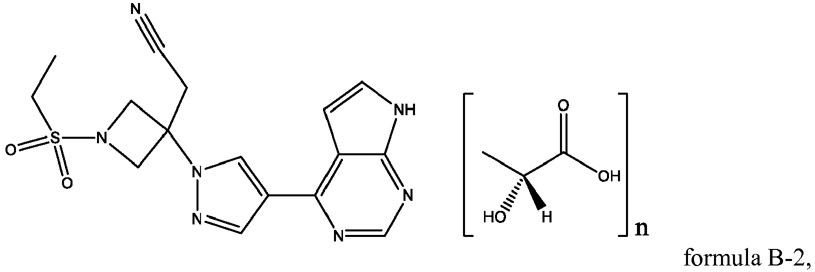 wherein n is in the range of from 0.8 to 1.2, preferably of from 0.9 to 1.1, even more preferably of from 0.95 to 1.05 and most preferably n is 1.0.

In another embodiment the invention relates to a co-crystal of baricitinib with lactic acid, preferably *L*-lactic acid, characterized by having a PXRD comprising reflections at 2-Theta angles of:
(7.2 ± 0.2)°, (9.0 ± 0.2)° and (10.7 ± 0.2)°; or
(7.2 ± 0.2)°, (9.0 ± 0.2)°, (10.7 ± 0.2)° and (19.8 ± 0.2)°; or
(7.2 ± 0.2)°, (9.0 ± 0.2)°, (10.7 ± 0.2)°, (17.7 ± 0.2)° and (19.8 ± 0.2)°; or
(7.2 ± 0.2)°, (9.0 ± 0.2)°, (10.7 ± 0.2)°, (12.2 ± 0.2)°, (17.7 ± 0.2)° and (19.8 ± 0.2)°; or
(7.2 ± 0.2)°, (9.0 ± 0.2)°, (10.7 ± 0.2)°, (12.2 ± 0.2)°, (14.5 ± 0.2)°, (17.7 ± 0.2)° and (19.8 ± 0.2)°; or
(7.2 ± 0.2)°, (9.0 ± 0.2)°, (10.7 ± 0.2)°, (11.3 ± 0.2)°, (12.2 ± 0.2)°, (14.5 ± 0.2)°, (17.7 ± 0.2)° and (19.8 ± 0.2)°; or
(7.2 ± 0.2)°, (9.0 ± 0.2)°, (10.7 ± 0.2)°, (11.3 ± 0.2)°, (12.2 ± 0.2)°, (14.5 ± 0.2)°, (17.7 ± 0.2)°, (19.4 ± 0.2)° and (19.8 ± 0.2)°; or
(7.2 ± 0.2)°, (9.0 ± 0.2)°, (10.7 ± 0.2)°, (11.3 ± 0.2)°, (12.2 ± 0.2)°, (13.6 ± 0.2)°, (14.5 ± 0.2)°, (17.7 ± 0.2)°, (19.4 ± 0.2)° and (19.8 ± 0.2)°,
when measured at RT with Cu-Kalpha_{1,2} radiation having a wavelength of 0.15419 nm.

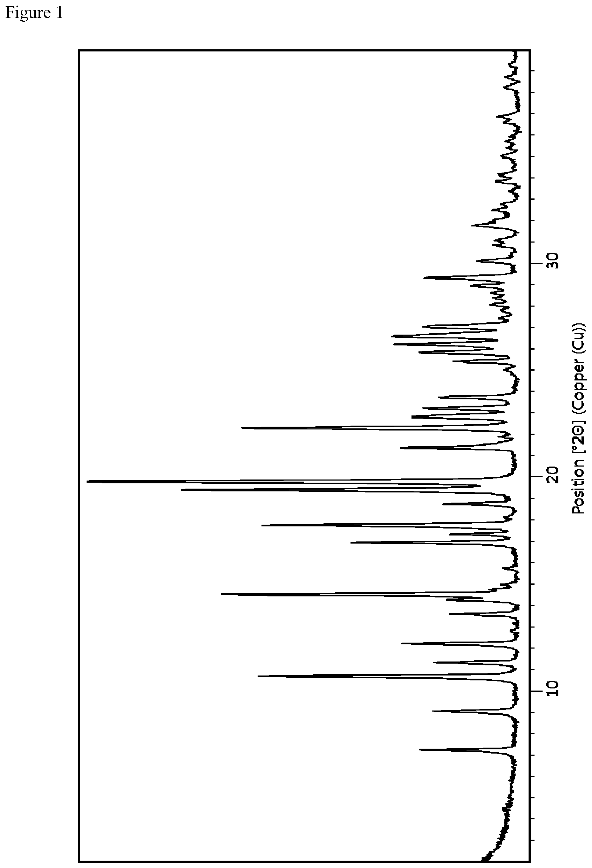

In yet another embodiment the invention relates to a co-crystal of baricitinib with lactic acid, preferably *L*-lactic acid, characterized by having a PXRD essentially the same as shown in figure 1 of the present invention, when measured at RT with Cu-Kalpha_{1,2} radiation having a wavelength of 0.15419 nm.

In a further embodiment, the present invention relates to a co-crystal of baricitinib with lactic acid, preferably *L*-lactic acid, characterized by having a FTIR spectrum comprising peaks at wavenumbers of:
(3482 ± 2) cm⁻¹, (1689 ± 2) cm⁻¹ and (1327 ± 2) cm⁻¹ or;
(3482 ± 2) cm⁻¹, (1689 ± 2) cm⁻¹, (1588 ± 2) cm⁻¹ and (1327 ± 2) cm⁻¹; or
(3482 ± 2) cm⁻¹, (1689 ± 2) cm⁻¹, (1588 ± 2) cm⁻¹, (1327 ± 2) cm⁻¹ and (1139 ± 2) cm⁻¹; or
(3482 ± 2) cm⁻¹, (3115 ± 2) cm⁻¹, (1689 ± 2) cm⁻¹, (1588 ± 2) cm⁻¹, (1327 ± 2) cm⁻¹ and (1139 ± 2) cm⁻¹; or
(3482 ± 2) cm⁻¹, (3115 ± 2) cm⁻¹, (1689 ± 2) cm⁻¹, (1588 ± 2) cm⁻¹, (1451 ± 2) cm⁻¹, (1327 ± 2) cm⁻¹ and (1139 ± 2) cm⁻¹; or
(3482 ± 2) cm⁻¹, (3115 ± 2) cm⁻¹, (1689 ± 2) cm⁻¹, (1588 ± 2) cm⁻¹, (1451 ± 2) cm⁻¹, (1327 ± 2) cm⁻¹, (1278 ± 2) cm⁻¹ and (1139 ± 2) cm⁻¹; or
(3482 ± 2) cm⁻¹, (3115 ± 2) cm⁻¹, (2982 ± 2) cm⁻¹, (1689 ± 2) cm⁻¹, (1588 ± 2) cm⁻¹, (1451 ± 2) cm⁻¹, (1327 ± 2) cm⁻¹, (1278 ± 2) cm⁻¹ and (1139 ± 2) cm⁻¹; or
(3482 ± 2) cm⁻¹, (3115 ± 2) cm⁻¹, (2982 ± 2) cm⁻¹, (2855 ± 2) cm⁻¹, (1689 ± 2) cm⁻¹, (1588 ± 2) cm⁻¹, (1451 ± 2) cm⁻¹, (1327 ± 2) cm⁻¹, (1278 ± 2) cm⁻¹ and (1139 ± 2) cm⁻¹; or
when measured at RT with a diamond ATR cell.

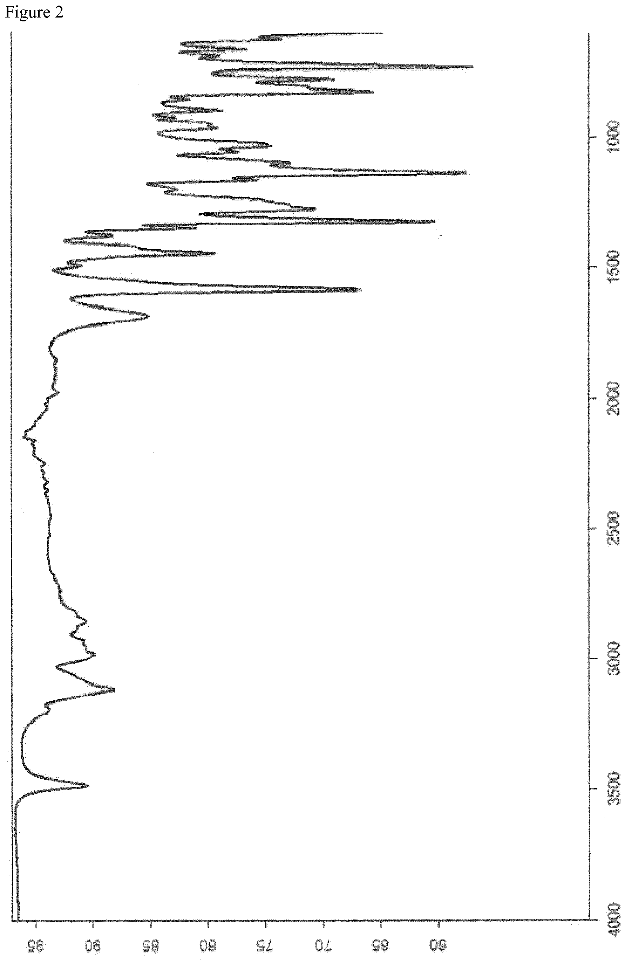

In yet another embodiment, the present invention relates to a co-crystal of baricitinib with lactic acid, preferably *L*-lactic acid, characterized by having a FTIR spectrum essentially the same as shown in figure 2 of the present invention, when measured at RT with a diamond ATR cell.

In a further embodiment, the present invention relates to a co-crystal of baricitinib with lactic acid, preferably *L*-lactic acid, characterized by having a Raman spectrum comprising peaks at wavenumbers of:
(2931 ± 3) cm⁻¹, (1595 ± 3) cm⁻¹ and (1283 ± 3) cm⁻¹; or
(3145 ± 3) cm⁻¹, (2931 ± 3) cm⁻¹, (1595 ± 3) cm⁻¹ and (1283 ± 3) cm⁻¹; or
(3145 ± 3) cm⁻¹, (2931 ± 3) cm⁻¹, (2252 ± 3) cm⁻¹, (1595 ± 3) cm⁻¹ and (1283 ± 3) cm⁻¹; or
(3145 ± 3) cm⁻¹, (2931 ± 3) cm⁻¹, (2252 ± 3) cm⁻¹, (1595 ± 3) cm⁻¹, (1283 ± 3) cm⁻¹ and (1193 ± 3) cm⁻¹; or
(3145 ± 3) cm⁻¹, (2931 ± 3) cm⁻¹, (2252 ± 3) cm⁻¹, (1595 ± 3) cm⁻¹, (1556 ± 3) cm⁻¹, (1283 ± 3) cm⁻¹ and (1193 ± 3) cm⁻¹; or
(3145 ± 3) cm⁻¹, (2931 ± 3) cm⁻¹, (2252 ± 3) cm⁻¹, (1595 ± 3) cm⁻¹, (1556 ± 3) cm⁻¹, (1478 ± 3) cm⁻¹, (1283 ± 3) cm⁻¹ and (1193 ± 3) cm⁻¹; or
(3145 ± 3) cm⁻¹, (2931 ± 3) cm⁻¹, (2252 ± 3) cm⁻¹, (1595 ± 3) cm⁻¹, (1556 ± 3) cm⁻¹, (1478 ± 3) cm⁻¹, (1283 ± 3) cm⁻¹, (1193 ± 3) cm⁻¹ and (825 ± 3) cm⁻¹; or
(3145 ± 3) cm⁻¹, (2931 ± 3) cm⁻¹, (2252 ± 3) cm⁻¹, (1595 ± 3) cm⁻¹, (1556 ± 3) cm⁻¹, (1478 ± 3) cm⁻¹, (1283 ± 3) cm⁻¹, (1193 ± 3) cm⁻¹, (905 ± 3) cm⁻¹ and (825 ± 3) cm⁻¹,
when measured at RT and a wavelength of 785 nm.

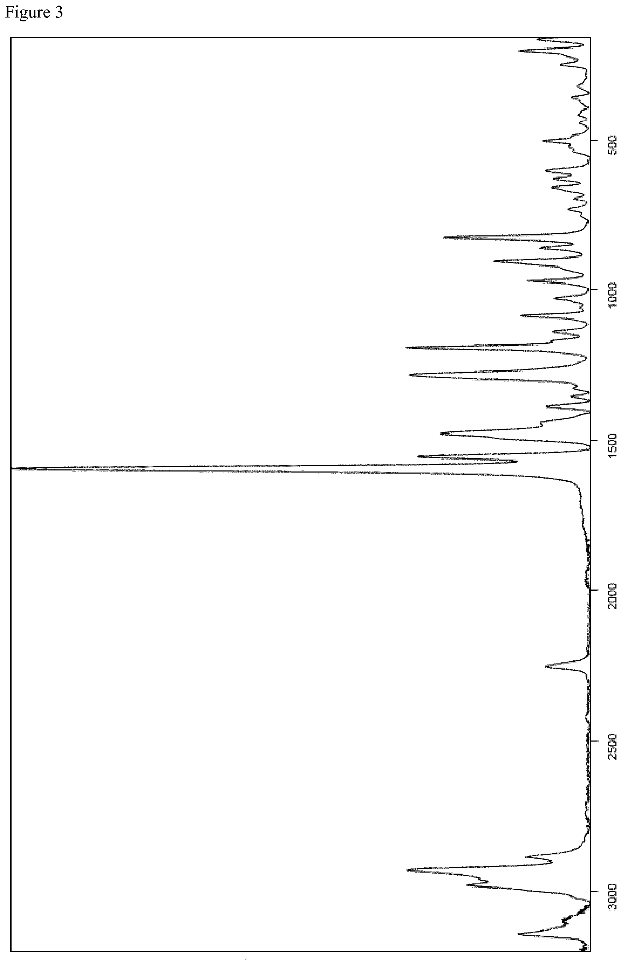

In yet another embodiment, the present invention relates to a co-crystal of baricitinib with lactic acid, preferably *L*-lactic acid, characterized by having a Raman spectrum essentially the same as shown in figure 3 of the present invention, when measured at RT and a wavelength of 785 nm.

In a further embodiment, the present invention relates to a co-crystal of baricitinib with lactic acid, preferably *L*-lactic acid, characterized by exhibiting monoclinic unit cells having space group P2₁ with the following parameters:
a = 6.8810
b= 19.486
c= 15.5180
alpha = 90°
beta = 93.636°
gamma = 90°
when measured with single crystal X-ray diffraction at (173 ± 2) K with Cu-Kalpha_{1,2} radiation having a wavelength of 1.54184 Angstrom.

In one embodiment, the present invention relates to a co-crystal of baricitinib with lactic acid, preferably *L*-lactic acid, characterized in that the co-crystal is anhydrous.

In another embodiment, the present invention relates to a co-crystal of baricitinib with lactic acid, preferably *L*-lactic acid, characterized in that the co-crystal is non-solvated.

In another embodiment, the present invention relates to a co-crystal of baricitinib with lactic acid, preferably *L*-lactic acid, characterized by having a DSC curve comprising an endothermic peak, preferably a single endothermic peak, having an onset temperature of (168 ± 1)°C, when measured with DSC at a heating rate of 10 K/min.

In a further embodiment, the present invention relates to a co-crystal of baricitinib with lactic acid, preferably *L*-lactic acid, characterized by having a DSC curve comprising an endothermic peak, preferably a single endothermic peak, having a peak temperature of (170 ± 1)°C, when measured with DSC at a heating rate of 10 K/min.

Thermal analysis by DSC suggests that the baricitinib lactic acid co-crystal of the present invention is thermally stable e.g. does not undergo phase transformations or decomposition until it melts at about 170 °C.

Hence, the baricitinib lactic acid co-crystal of the present invention is sufficiently stable to be used in and for the preparation of a pharmaceutical composition.

In another aspect, the present invention relates to a composition comprising the baricitinib lactic acid co-crystal of the present invention as defined in any of the embodiments described above, said composition being essentially free of any other solid-state form of baricitinib. For example, a composition comprising the baricitinib lactic acid co-crystal of the present invention comprises at most 20 weight%, preferably at most 10 weight%, more preferably at most 5 weight%, 4 weight%, 3 weight%, 2 weight% or 1 weight% of any other solid form of baricitinib, based on the weight of the composition. Preferably, the any other solid form of baricitinib is form A disclosed in WO 2015/166434 A1, WO 2016/141891 A1, and CN105693731 A. Form A of baricitinib has a PXRD comprising amongst others characteristic reflections at 2-Theta angles of (16.32 ± 0.2)° and (20.40 ± 0.2)°, when measured at a temperature in the range of from 20 to 30 °C with Cu-Kalpha_{1,2} radiation having a wavelength of 0.15419 nm. Therefore, the absence of reflections at 2-Theta angles of (16.32 ± 0.2)° and (20.40 ± 0.2)° in the PXRD confirms the absence of baricitinib form A in the composition.

Hence, in a preferred embodiment, the present invention relates to a composition comprising the baricitinib lactic acid co-crystal of the present invention as defined in any of the embodiments described above, said composition having a PXRD comprising no reflections at 2-Theta angles of (16.32 ± 0.2)° and (20.40 ± 0.2)°, when measured at a temperature in the range of from 20 to 30 °C with Cu-Kalpha_{1,2} radiation having a wavelength of 0.15419 nm.

In a further aspect, the present invention relates to a process for the preparation of the baricitinib lactic acid co-crystal of the present invention or the composition comprising the baricitinib lactic acid co-crystal as defined in any one of the aspects and their corresponding embodiments described above comprising:
(a) preparing a solution comprising baricitinib, lactic acid and at least one suitable solvent,
(b) evaporating the solvent at ambient conditions;
(c) optionally, seeding the solution obtained in (a) or (b) with baricitinib lactic acid co-crystals according to the present invention;
(d) optionally, separating at least a part of the crystals obtained in (b) or (c) from the mother liquor;
(e) optionally, washing the isolated crystals obtained in (d); and
(f) optionally, drying the crystals obtained in any one of steps (b) to (e).

Baricitinib can for example be prepared according to the procedure provided in example 1 or example 70 of WO 2009/114512 A1 and optionally transformation into its free base form can be effected.

The solution of baricitinib in a solvent or solvent mixture may be prepared within a concentration range of from about 2 to 10 g/L, most preferably the baricitinib concentration of the solution prepared is 4 g/L. The molar ratio of baricitinib and lactic acid applied may be in the range of from 1.0 : 0.8 to 1.0 to 1.2, preferably of from 1.0 : 0.9 to 1.0 to 1.1, even more preferably of from 1.0 to 0.95 to 1.0 to 1.05 and most preferably the molar ratio is 1.0 : 1.0. The at least one suitable solvent is preferably methylethylketone (MEK). The solution may be prepared at RT or at elevated temperature, preferably the solution is prepared at RT. Preferably the solution for step a) is a solution of baricitinib and lactic acid in methylethylketone.

Optionally, baricitinib lactic acid co-crystals may be added as seeds in order to promote crystallization and/or to control particle size distribution. The amount of seed crystals employed may range from about 1 to 20 weight%, preferably from about 1 to 10 weight% and most preferably from about 1 to 5 weight%, based on the weight of applied baricitinib starting material. Seed crystals may be prepared according to steps (a) to (b) of the above described procedure e.g. according to the procedure disclosed in example 1 of the present invention.

The solvent or solvent mixture may be partially removed in a periode of about 1 to 5 days, preferentially it is removed within 3 days. The solventor solvent mixture may be partially removed at RT or elevated temperature, preferentially it is removed at RT. The evaporation process may be operated at reduced or ambient pressure, preferentially it is operated at ambient pressure.

Once the baricitinib lactic acid co-crystal of the present invention is obtained or preferably obtained in essentially pure form, at least a part of the crystals may be optionally separated from the mother liquor. Preferably, the crystals are separated from their mother liquor by any conventional method such as filtration, centrifugation, solvent evaporation or decantation, more preferably by filtration or centrifugation and most preferably by filtration.

Optionally, in a further step the isolated crystals are washed with at least one solvent, e.g. aliphatic ether solvents selected from the group consisting of diisopropyl ether, tert-butylmethyl ether and diethyl ether or any mixtures thereof. Preferably, diisopropyl ether and/or tert-butylmethyl ether are used.

The obtained crystals may then optionally be dried. Drying may be performed at a temperature in the range of from about 20 to 80 °C, preferably in the range of from about 20 to 40 °C and most preferably drying is performed at RT. Drying may be performed for a period in the range of from about 1 to 72 hours, preferably of from about 2 to 48 hours, more preferably of from about 4 to 24 hours and most preferably of from about 6 to 18 hours. Drying may be performed at ambient pressure and/ or under reduced pressure. Preferably, drying is performed at a pressure of about 100 mbar or less, more preferably of about 50 mbar or less and most preferably of about 30 mbar or less, for example a vacuum of about 25 mbar is applied for drying.

In a further aspect, the present invention relates to the use of the baricitinib lactic acid co-crystal of the present invention or the composition comprising the baricitinib lactic acid co-crystal as defined in any one of the aspects and their corresponding embodiments described above for the preparation of a pharmaceutical composition.

In a further aspect, the present invention relates to a pharmaceutical composition comprising the baricitinib lactic acid co-crystal of the present invention or the composition comprising the baricitinib lactic acid co-crystal as defined in any one of the aspects and their corresponding embodiments described above, preferably in an effective and/or predetermined amount, and at least one pharmaceutically acceptable excipient.

Preferably, the predetermined and/or effective amount of the baricitinib lactic acid co-crystal of the present invention is in the range of from 1 to 20 mg calculated as baricitinib. For example the predetermined and/or effective amount of the baricitinib lactic acid co-crystal of the present invention is 2 mg, 4 mg, 6 mg, 8mg, 10 mg or 20 mg, preferably 2 mg, 4 mg or 5mg and most preferably 2 or 4 mg calculated as baricitinib.

The at least one pharmaceutically acceptable excipient, which is comprised in the pharmaceutical composition of the present invention, is preferably selected from the group consisting of fillers, diluents, binders, disintegrants, lubricants, glidants and combinations thereof. Preferably, the at least one pharmaceutically acceptable excipient is selected from the group consisting of lactose monohydrate, microcrystalline cellulose, povidone, croscarmellose sodium, magnesium stearate, mannitol and combinations thereof. All of these pharmaceutically acceptable excipients can be comprised by the pharmaceutical composition of the present invention. Optionally, the pharmaceutical composition comprises lactic acid.

Preferably, the pharmaceutical composition of the present invention as described above is an oral solid dosage form.

In a particular embodiment, the pharmaceutical composition of the present invention as describe above is a tablet, preferably a film-coated tablet comprising a tablet core and a coating.

The tablet or tablet core may be prepared by mixing the baricitinib lactic acid co-crystal with at least one excipient such as fillers, diluents, binders, disintegrants, lubricants, glidants or combinations thereof followed by compressing the mixture. Optionally, a dry granulation step is performed before compression. Preferably, the tablet core is subsequently coated with a film-coat, whereat non-limiting examples of coatings include polyvinylalcohol-based, hydroxyethylcellulose, hydroxypropylmethylcellulose, carboxymethylcellulose sodium polyethylene glycol 4000 and cellulose acetate phthalate coatings. Methods of preparing such tablets, tablet cores and film-coated tablets are well known in the pharmaceutical arts.

In another particular embodiment, the pharmaceutical composition of the present invention as describe above is a capsule. In a further embodiment, the capsule shell is a gelatin shell or a hydroxypropylmethylcellulose (HPMC) shell.

In another embodiment of the present invention the dosage form of the present invention is packed by a suitable packaging material. The packaging material preferably reduces or prevents water exchange between the pharmaceutical composition of the present invention and the environment. For example, if the dosage forms are tablets or capsules, suitable blister pack materials can be used. The blister pack may comprise a cavity or pocket, preferably containing a thermoformed plastic. This usually has as a backing a lidding seal containing an aluminum and/or plastic foil. Further, if the composition is in form of a granulate, suitable sachets can be used.

In a particularly preferred embodiment the pharmaceutical composition or the dosage form of the present invention is packed by a material having a water vapor permeability of 0.001 to 0.15 g/m²/day at 38°C/5%/90% RH, preferably of 0.01 to 0.12 g/m²/day at 38°C/5%/90% RH, in particular 0.05 to 0.10 g/m2/day at 38°C/5%/90% RH, wherein said water vapor permeability is determined according to ASTM F1249-13. Preferably, a Permatran-W Model 3/33 device is used. The measurement is preferably carried out at 38°C. Further, preferably the humidity in the dry chamber is 5% relative humidity (=RH), whereas the humidity in the wet chamber is 90% RH.

In a preferred embodiment the packaging material can preferably be selected from polyvinylchloride (PVC), polyvinylidene chloride (PVDC), polyethylene (PE), polypropylene (PP), polyethylene terephthalate (PET) polystyrol (PS), polyamide and alumina or combinations thereof. Alu/Alu blisters are particularly preferred.

In a preferred embodiment the packing material comprises layered sheets, which can be thermoformed, containing one or more layers. In a preferred embodiment the packing material can be a composite material, e.g. co-extruded composite material, e.g. a polyamide-alumina-polyvinyl chloride composite material, which is also referred to as Nylon®-Alu-PVC.

In a preferred embodiment the packaging material has a thickness of 1 µm to 1 mm. In case of a blister pack the thermoformed plastic pocket preferably has a thickness of 100 to 1000 µm, more preferably of 150 to 800 µm. Further, the backing foil usually has a thickness of 10 to 150 µm, more preferably of 15 to 100 µm.

In a further aspect, the present invention relates to the baricitinib lactic acid co-crystal, the composition comprising the baricitinib lactic acid co-crystal or the pharmaceutical composition comprising the baricitinib lactic acid co-crystal as defined in any one of the above described aspects and their corresponding embodiments for use as a medicament.

In yet another aspect, the present invention relates to the baricitinib lactic acid co-crystal, the composition comprising the baricitinib lactic acid co-crystal or the pharmaceutical composition comprising the baricitinib lactic acid co-crystal as defined in any one of the above described aspects and their corresponding embodiments for use in the treatment of a JAK-associated disease. This includes any disease, disorder or condition that can be prevented, ameliorated, or cured by modulating JAK activity.

Examples of JAK-associated diseases include diseases involving the immune system including, for example, organ transplant rejection (e.g., allograft rejection and graft versus host disease).

Further examples of JAK-associated diseases include autoimmune diseases such as multiple sclerosis, rheumatoid arthritis, juvenile arthritis, psoriatic arthritis, type I diabetes, systemic lupus erythematosus (SLE), psoriasis, inflammatory bowel disease, ulcerative colitis, Crohn's disease, myasthenia gravis, immunoglobulin nephropathies, autoimmune thyroid disorders, and the like. In some embodiments, the autoimmune disease is an autoimmune bullous skin disorder such as pemphigus vulgaris (PV) or bullous pemphigoid (BP). Further examples of JAK-associated diseases include allergic conditions such as asthma, food allergies, atopic dermatitis and rhinitis. Further examples of JAK-associated diseases include viral diseases such as Epstein Barr Virus (EBV), Hepatitis B, Hepatitis C, HIV, HTLV 1, Varicella-Zoster Virus (VZV) and Human Papilloma Virus (HPV).

Further examples of JAK-associated diseases or conditions include skin disorders such as psoriasis (for example, psoriasis vulgaris), atopic dermatitis, skin rash, skin irritation, skin sensitization (e.g., contact dermatitis or allergic contact dermatitis). For example, certain substances including some pharmaceuticals when topically applied can cause skin sensitization. In some embodiments, co-administration or sequential administration of at least one JAK inhibitor of the invention together with the agent causing unwanted sensitization can be helpful in treating such unwanted sensitization or dermatitis. In some embodiments, the skin disorder is treated by topical administration of at least one JAK inhibitor of the invention.

JAK-associated diseases can further include those characterized by expression of a mutant JAK2 such as those having at least one mutation in the pseudo-kinase domain (e.g., JAK2V617F). JAK-associated diseases can further include myeloproliferative disorders (MPDs) such as polycythemia vera (PV), essential thrombocythemia (ET), myelofibrosis with myeloid metaplasia (MMM), chronic myelogenous leukemia (CML), chronic myelomonocytic leukemia (CMML), hypereosinophilic syndrome (HES), systemic mast cell disease (SMCD), and the like. In some embodiments, the myeloproliferative disorder is primary myelofibrosis (PMF) or post polycythemia vera/essential thrombocythemia myelofibrosis (Post-PV/ET MF).

Further JAK-associated diseases include inflammation and inflammatory diseases. Example inflammatory diseases include inflammatory diseases of the eye (e.g., iritis, uveitis, scleritis, conjunctivitis, or related disease), inflammatory diseases of the respiratory tract (e.g., the upper respiratory tract including the nose and sinuses such as rhinitis or sinusitis or the lower respiratory tract including bronchitis, chronic obstructive pulmonary disease, and the like), inflammatory myopathy such as myocarditis, and other inflammatory diseases.

The the baricitinib lactic acid co-crystal described herein can further be used to treat ischemia reperfusion injuries or a disease or condition related to an inflammatory ischemic event such as stroke or cardiac arrest. The JAK inhibitors described herein can further be used to treat anorexia, cachexia, or fatigue such as that resulting from or associated with cancer. The JAK inhibitors described herein can further be used to treat restenosis, sclerodermitis, or fibrosis. The JAK inhibitors described herein can further be used to treat conditions associated with hypoxia or astrogliosis such as, for example, diabetic retinopathy, cancer, or neurodegeneration.

The the baricitinib lactic acid co-crystal described herein can further be used to treat other inflammatory diseases such as systemic inflammatory response syndrome (SIRS) and septic shock.

The the baricitinib lactic acid co-crystal described herein, as well as other JAK inhibitors capable of influencing IL-6/STAT3 signalling, can further be used to treat inflammation-associated proliferative diseases. Inflammation has been shown to be linked to the development of certain types of cancers. For example, patients suffering from inflammatory bowel disease such as ulcerative colitis have been shown to have a much higher risk of developing colorectal cancer. These types of inflammation-linked cancers have been termed colitis-associated cancer (CAC). Several studies have shown that the IL-6/STAT3 signaling is involved in promoting CAC.

In a particular preferred embodiment, the invention relates to the baricitinib lactic acid co-crystal, the composition comprising the baricitinib lactic acid co-crystal or the pharmaceutical composition comprising the baricitinib lactic acid co-crystal as defined in any one of the above described aspects and their corresponding embodiments for use in the treatment and/or prophylaxis of rheumatoid arthritis, atopic dermatitis, systemic lupus erythematosus (SLE), psoriatic arthritis, and psoriasis.

The the baricitinib lactic acid co-crystal described herein can further be used for the preparation of compostion for treatment of alopecia areata.

### EXAMPLES

The following non-limiting examples are illustrative for the disclosure and are not to be construed as to be in any way limiting for the scope of the invention.

### Example 1: Preparation of the baricitinib lactic acid co-crystal of the present invention

A solution of equimolar amounts of baricitinib (40 mg) and *L*-lactic acid (9.7 mg) in 10 mL methylethylketone (MEK) was prepared. Then the solvent was slowly evaporated in a crystallization dish (h=30mm d= 50mm) at ambient conditions (T=20°C, p= 1013mbar rH= 30%). After three days, clear colorless needle-shaped crystals grew in the solution. The crystals were separated and dried on filter paper at 40°C. Further analysis, in particular the structural determination by single crystal X-ray in example 5, determined that a co-crystal between baricitinib and lactic acid had formed, wherein both molecules were present as neutral species.

### Example 2: Powder X-ray diffraction

The co-crystals from experiment 1 were investigated by powder X-ray diffraction, which was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta. Thus, the diffraction peak of the baricitinib lactic acid co-crystal of the present invention which appears at 7.2° 2-Theta in figure 1 can appear in the range of from 7.0 to 7.4° 2-Theta, preferably in the range of from 7.1 to 7.3° 2-Theta on most X-ray diffractometers under standard conditions.

A representative diffractogram of the baricitinib lactic acid co-crystal according to the present invention is displayed in figure 1 and the corresponding reflection list (peak list) from 2 to 30° 2-Theta is provided in table 1 below.

**Table 1: Reflection (peak) positions of the baricitinib lactic acid co-crystal according to the present invention in the range of from 2 to 30° 2-Theta; A typical precision of the 2-Theta values is in the range of ± 0.2° 2-Theta, preferably of ± 0.1 ° 2-Theta.**

| **Reflection position [° 2-Theta]** | **Reflection position [° 2-Theta]** | **Reflection position [° 2-Theta]** | **Reflection position [° 2-Theta]** |
|---|---|---|---|
| 7.2 | 15.7 | 22.8 | 27.8 |
| 9.0 | 16.9 | 23.2 | 28.1 |
| 10.7 | 17.3 | 23.7 | 28.3 |
| 11.3 | 17.7 | 25.4 | 28.6 |
| 13.6 | 18.7 | 25.8 | 28.9 |
| 14.3 | 19.4 | 26.2 | 29.3 |
| 14.5 | 19.8 | 26.6 | |
| 14.8 | 21.4 | 27.0 | |
| 15.0 | 22.3 | 27.4 | |

### Example 3: Fourier transform infrared spectroscopy

The baricitinib lactic acid co-crystal according to the present invention was investigated by FTIR spectroscopy. The FTIR spectrum was recorded (obtained) on a MKII Golden Gate™ Single Reflection Diamond ATR cell with a Bruker Tensor 27 FTIR spectrometer with 4 cm⁻¹ resolution at RT. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of from about ± 2 cm⁻¹. Thus, the infrared peak of the baricitinib lactic acid co-crystal according to the present invention at 3482 cm⁻¹ can appear between 3480 and 3484 cm⁻¹ on most infrared spectrometers under standard conditions.

A representative FTIR spectrum of the baricitinib lactic acid co-crystal according to the present invention is displayed in figure 2 and the corresponding peak list is provided in table 2 below.

**Table 2: FTIR peak list of the baricitinib lactic acid co-crystal according to the present invention; a typical precision of the wavenumbers is in the range of ± 2 cm⁻¹.**

| **Wavenumber [cm⁻¹]** | **Wavenumber [cm⁻¹]** | **Wavenumber [cm⁻¹]** |
|---|---|---|
| 3482 | 1327 | 930 |
| 3115 | 1278 | 902 |
| 2982 | 1207 | 860 |
| 2855 | 1168 | 829 |
| 1689 | 1139 | 782 |
| 1588 | 1102 | 734 |
| 1499 | 1060 | 695 |
| 1451 | 1038 | 667 |
| 1383 | 968 | 630 |
| 1353 | | |

### Example 4: Raman spectroscopy

The baricitinib lactic acid co-crystal according to the present invention was investigated by Raman spectroscopy. Raman spectrum was recorded at a Bruker Bravo Analyzer equipped with a Duo LaserTM (Popt < 100mW @ λ = 700 - 1100nm) and a CCD detector. Data was collected in a spectral range from 3200 cm-1 to 300 cm-1 with a spectral resolution of 10 cm-1 to 12 cm-1. Sequentially Shifted Excitation (SSETM) was used as fluorescence mitigation technique. Thus, the peak of the baricitinib lactic acid co-crystal of the present invention at 1595 cm⁻¹ can appear between 1598 and 1592 cm⁻¹, preferably between 1597 and 1593 cm⁻¹ on most Raman spectrometers under standard conditions.

A representative Raman spectrum of the baricitinib lactic acid co-crystal according to the present invention is displayed in figure 3 and the corresponding peak list is provided in table 3 below.

**Table 3: Raman peak list of the baricitinib lactic acid co-crystal according to the present invention; a typical precision of the wavenumbers is in the range of from ± 1 to ± 3 cm⁻¹.**

| **Wavenumber [cm⁻¹]** | **Wavenumber [cm⁻¹]** | **Wavenumber [cm⁻¹]** | **Wavenumber [cm⁻¹]** |
|---|---|---|---|
| 3145 | 1389 | 905 | 505 |
| 2981 | 1355 | 862 | 445 |
| 2931 | 1283 | 825 | 418 |
| 2888 | 1193 | 733 | 361 |
| 2252 | 1141 | 695 | 322 |
| 1595 | 1087 | 659 | 251 |
| 1556 | 1028 | 631 | 205 |
| 1478 | 971 | 604 | 167 |

### Example 5: Single crystal X-ray diffraction

Intensity data for the crystal structure of the baricitinib lactic acid co-crystal of the present invention were collected with Mo (lambda = 0.71073 Angstrom) radiation on an Oxford Diffraction Gemini-R Ultra diffractometer at 173 K. The structure was solved using the direct methods procedure in SHELXT and refined by full-matrix least squares on F² using SHELXL-2014. All H atoms were located in difference maps. H atoms bonded to O or N atoms were refined with restrained distances [O-H = 0.84 Å; N-H = 0.88 Å) and their Uᵢₛₒ parameters were refined freely. The H atoms bonded to C atoms were refined using a riding model with Uᵢₛₒ set at 1.2U_{eq} or 1.5U_{eq} of the parent C atom. The carboxylic acid function of lactic acid was protonated.

### Example 6: Differential scanning calorimetry (DSC)

The baricitinib lactic acid co-crystal according to the present invention was investigated by DSC, which was performed with a DSC 7 (Perkin-Elmer, Norwalk, Ct., USA) using a Pyris 2.0 software. Approximately 4± 0.0005 mg sample (using a UM3 ultramicrobalance, Mettler, Greifensee, CH) were weighed into Al-Pans (25 µl) and sealed with a cover, which was perforated by a needle. Dry nitrogen was used as the purge gas (purge: 20 ml/min). Heating rate: 10 K/min.

The DSC curve shows a single endothermic peak with an onset temperature of about 168 °C and a peak temperature of about 170 °C, which is due to the melting of the sample. The anhydrous and non-solvated nature of the co-crystal and its excellent thermal stability are evidenced by the fact that neither phase changes nor desolvation events are detectable until the sample melts.

### Example 7: Thermogravimetric Analysis (TGA)

Thermogravimetric-system TGA-7, Pyris-Software für Windows NT, (Perkin-Elmer, Norwalk, Ct., USA), Platinum-sample holder (50 µl), Nitrogen as the purge gas (Sample purge: 20 ml min-1, balance purge: 40 ml*min-1). Heating rate: 10 K*min-1.

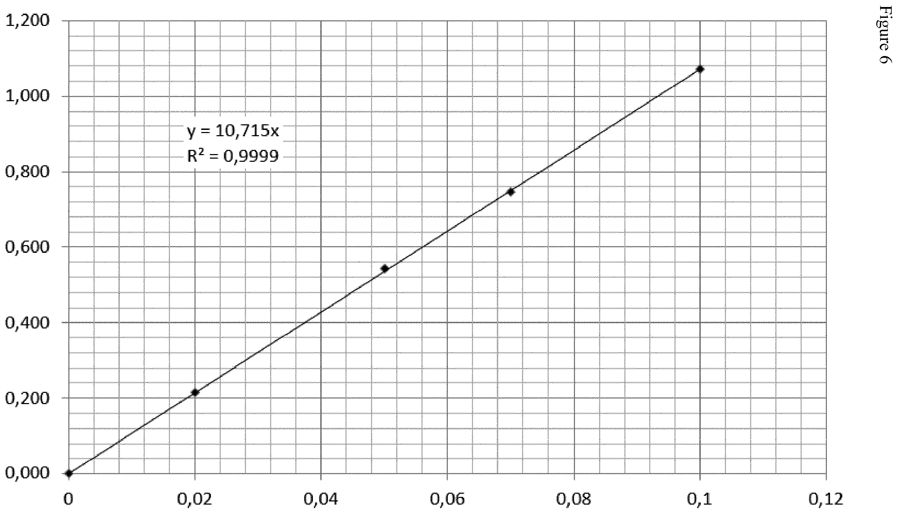

Between a temperature of 159 °C and 190 °C the lactic acid co-crystal is loosing about 18,2% of its weight, which is equivalent to about 0.91 molar equivalents of *L*-lactic acid. The curve is displayed in figure 6 together with a DSC featuring an onset of elevated heatflow at 169 °C and a peak maximum of 172 °C. The sample for both experiments was obtained by the procedure of example 9

### Example 8: Dissolution testing

Experiment 1: 5 mg of crystalline baricitinib free base Form A were stirred at room temperature in 100ml of water and showed no detectable dissolution. When the same molar amount of the baricitinib lactic acid co-crystal of the present invention was stirred at room temperature in the same amount of water, a detectable and significant amount dissolved within minutes.

Experiment 1 clearly indicated significant and faster dissolution of the barcitinib lactic acid co-crystal of the invention compared to pure cristallin baritinib form A. However, no quantitative data could be derived from this setup.

In order to obtain more quantitative data we designed a photometric method based on the fact that baricitinib in pH neutral solutions shows a UV absorption maximum at 310 nm. We used this to quantify baricitinib concentrations. Light absorption was measured with a Shimadzu UV-1800 double beam photometer at 310 nm in a 1cm quartz glass cuvette.

Standards with concentrations of 0.1 ; 0.07 ; 0.05 and 0.02 mmol/L baricitinib were prepared and measured to provide a suitable calibration curve (displayed in figure 6).

To counterbalance the acidifying effect of the lactic acid from the barcitinib lactic acid co-crystal, the experiments were performed in a phosphate buffer solution at a constant pH of 6.8 (HPO4-/HPO4²⁻ ; 0,01 mol/L). Furthermore, 0.01% Tween 80 was added to the buffer solution in order to avoid potential agglomeration during the dissolution test.

Experiment 2: 20 mg of crystalline baricitinib free base Form A or 24.85 mg of the baricitinib lactic acid co-crystal of the present invention were stirred in a closed vessel in 50mL of the buffer solution at 37°C. After 1, 5, 10, 15, 20, 30, 45 and 75 minutes filtered samples of the suspension were taken and diluted 1:4 with buffer solution to reach an absorption value within the calibration curve.

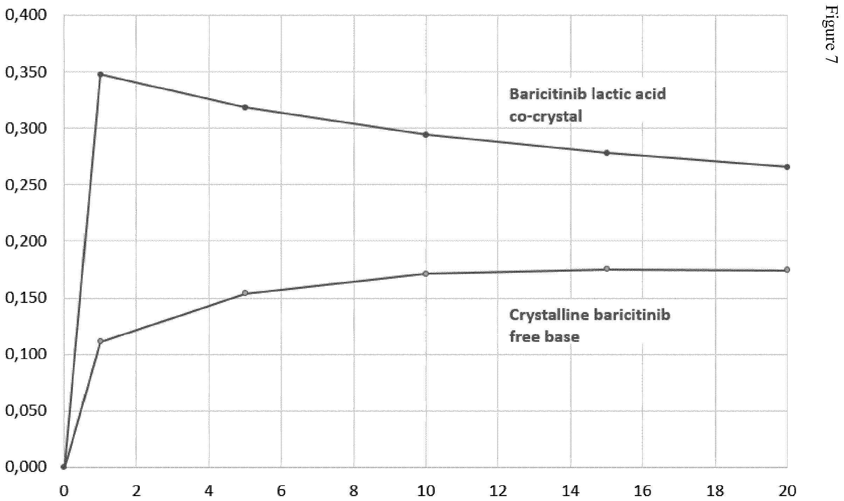

The outcome of experiment 2 is displayed in figure 7. The baricitinib lactic acid co-crystal of the current invention dissolved more quickly than pure cristalline baricitinib free base form A (compare for example the values at 1 minute, where the concentration of dissolved baricitinib is three time higher for the baricitinib lactic acid co-crystal when compred to baricitinib free base form A).

### Example 9: Alternative preparation of the baricitinib lactic acid co-crystal of the present invention

2.0 g of baricitinib and two molar equivalents of *L*-lactic acid (758mg) were mixed with 25mL ethanol in a closed stirring cylinder. The suspension was stirred for 24 hours at 750 rpm and 23°C. Then, the resulting white solid was filtered over vacuum and washed twice with cold ethanol. Drying at 40°C on a filter paper provided the pure baricitinib lactic acid co-crystal of the invention in 96,7% yield as confirmed by XRPD.

## Claims

1. A co-crystal of baricitinib with lactic acid.

2. The co-crystal of claim 1, wherein the molar ratio of baricitinib to lactic acid is 1:1.

3. The co-crystal of claims 1 or 2, wherein the lactic acid is *L*-lactic acid.

4. The co-crystal of claims 1 or 2, wherein the lactic acid is D-lactic acid.

5. The co-crystal of any one of claims 1 to 3 **characterized by** having the chemical structure according to formula B1 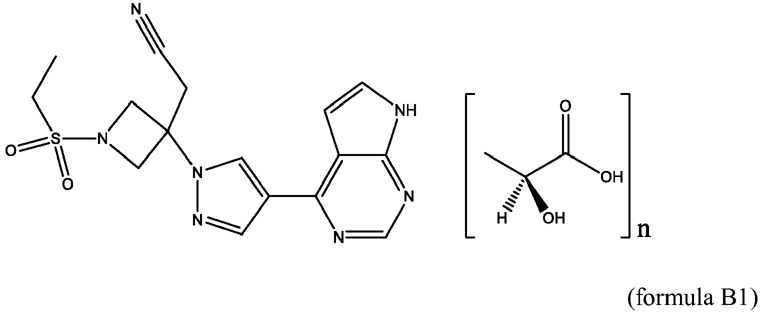 wherein n is is in the range of from 0.8 to 1.2.

6. The co-crystal of claim 1, 2 or 4 **characterized by** having the chemical structure according to formula B2 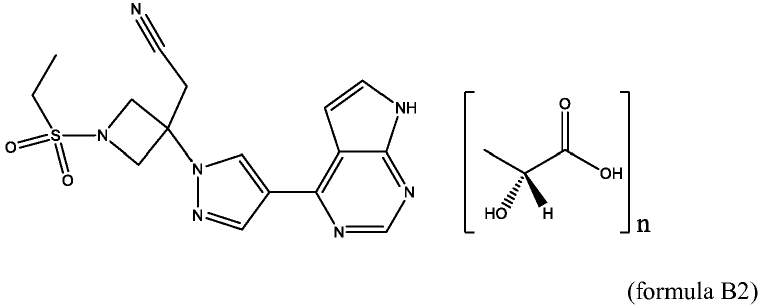 wherein n is is in the range of from 0.8 to 1.2.

7. The co-crystal according to any one of the preceding claims **characterized by** having a powder X-ray diffractogram comprising reflections at 2-Theta angles of (7.2 ± 0.2)°, (9.0 ± 0.2)° and (10.7 ± 0.2)°, when measured at a temperature in the range of from 20 to 30 °C with Cu-Kalpha_{1,2} radiation having a wavelength of 0.15419 nm.

8. The co-crystal according to any one of the preceding claims **characterized by** having a Fourier transform infrared spectrum comprising peaks at wavenumbers of (3482 ± 2) cm⁻¹, (1689 ± 2) cm⁻¹ and (1327 ± 2) cm⁻¹, when measured at a temperature in the range of from 20 to 30 °C with a diamond attenuated total reflection cell.

9. The co-crystal according to any one of the preceding claims **characterized by** having a Raman spectrum comprising peaks at wavenumbers of (2931 ± 3) cm⁻¹, (1595 ± 3) cm⁻¹ and (1283 ± 3) cm⁻¹, when measured at a temperature in the range of from 20 to 30 °C and a wavelength of 785 nm.

10. A composition comprising the co-crystal as defined in any one of the preceding claims **characterized by** having a powder X-ray diffractogram comprising no reflections at 2-Theta angles of (16.32 ± 0.2)° and (20.40 ± 0.2)°, when measured at a temperature in the range of from 20 to 30 °C with Cu-Kalpha_{1,2} radiation having a wavelength of 0.15419 nm.

11. Use of the co-crystal as defined in any one of claims 1 to 9 or the composition as defined in claim 10 for the preparation of a pharmaceutical composition.

12. A pharmaceutical composition comprising the co-crystal as defined in any one of claims 1 to 9 or the composition as defined in claim 10 and at least one pharmaceutically acceptable excipient.

13. The pharmaceutical composition according to claim 12, wherein the pharmaceutical composition is an oral solid dosage form.

14. The pharmaceutical composition of claim 13, wherein the oral solid dosage form is a tablet or a capsule.

15. The co-crystal as defined in any one of claims 1 to 9, the composition as defined in claim 10 or the pharmaceutical composition according to claim 12 to 14 for use in the treatment and/or prophylaxis of rheumatoid arthritis, atopic dermatitis, systemic lupus erythematosus (SLE), psoriatic arthritis and psoriasis.

## Patentansprüche

1. Co-Kristall von Baricitinib mit Milchsäure.

2. Co-Kristall nach Anspruch 1, wobei das molare Verhältnis von Baricitinib zu Milchsäure 1:1 beträgt.

3. Co-Kristall nach Anspruch 1 oder 2, wobei die Milchsäure L-Milchsäure ist.

4. Co-Kristall nach Anspruch 1 oder 2, wobei die Milchsäure D-Milchsäure ist.

5. Co-Kristall nach irgendeinem der Ansprüche 1 bis 3, **dadurch gekennzeichnet, dass** er die chemische Struktur gemäß Formel B1 aufweist 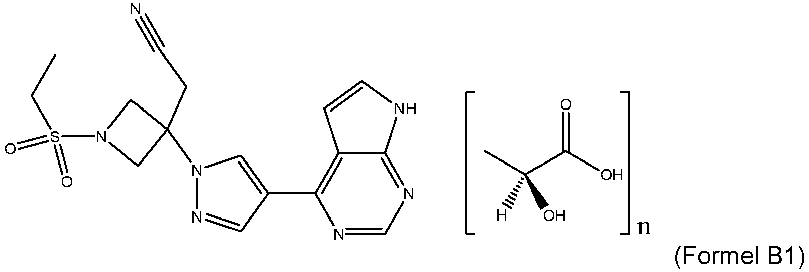 wobei n in dem Bereich von 0,8 bis 1,2 liegt.

6. Co-Kristall nach Anspruch 1, 2 oder 4, **dadurch gekennzeichnet, dass** er die chemische Struktur gemäß Formel B2 aufweist 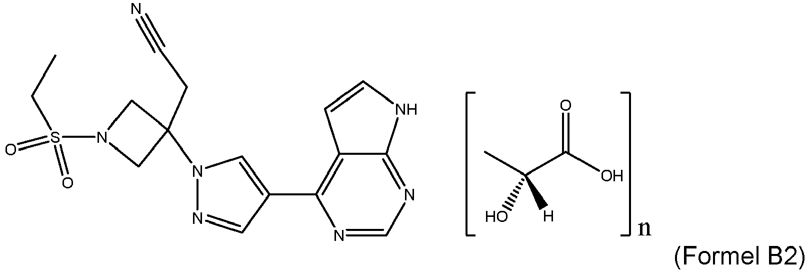 wobei n in dem Bereich von 0,8 bis 1,2 liegt.

7. Co-Kristall nach irgendeinem der vorhergehenden Ansprüche, **gekennzeichnet durch** ein Röntgenpulverdiffraktogramm mit Reflexen bei 2-Theta-Winkeln von (7,2 ± 0,2)°, (9,0 ± 0,2)° und (10,7 ± 0,2)°, gemessen bei einer Temperatur im Bereich von 20 bis 30 °C mit Cu-Kalpha_{1,2}-Strahlung mit einer Wellenlänge von 0,15419 nm.

8. Co-Kristall nach irgendeinem der vorhergehenden Ansprüche, **gekennzeichnet durch** ein Fourier-Transformations-Infrarotspektrum umfassend Peaks bei den Wellenzahlen (3482 ± 2) cm⁻¹, (1689 ± 2) cm⁻¹ und (1327 ± 2) cm⁻¹, gemessen bei einer Temperatur im Bereich von 20 bis 30 °C mit einer Diamant-Zelle mit abgeschwächter Totalreflexion.

9. Co-Kristall nach irgendeinem der vorhergehenden Ansprüche, **dadurch gekennzeichnet, dass** er ein Raman-Spektrum aufweist, das Peaks bei den Wellenzahlen (2931 ± 3) cm⁻¹, (1595 ± 3) cm⁻¹ und (1283 ± 3) cm⁻¹ umfasst, gemessen bei einer Temperatur im Bereich von 20 bis 30 °C und einer Wellenlänge von 785 nm.

10. Zusammensetzung, umfassend den in irgendeinem der vorhergehenden Ansprüche definierten Co-Kristall, **gekennzeichnet durch** ein Röntgenpulverdiffraktogramm, das keine Reflexe bei 2-Theta-Winkeln von (16,32 ± 0,2)° und (20,40 ± 0,2)° umfasst, gemessen bei einer Temperatur im Bereich von 20 bis 30 °C mit Cu-Kalpha_{1,2}-Strahlung mit einer Wellenlänge von 0,15419 nm.

11. Verwendung des in irgendeinem der Ansprüche 1 bis 9 definierten Co-Kristalls oder der in Anspruch 10 definierten Zusammensetzung zur Herstellung einer pharmazeutischen Zusammensetzung.

12. Pharmazeutische Zusammensetzung, umfassend den Co-Kristall, wie in irgendeinem der Ansprüche 1 bis 9 definiert, oder die Zusammensetzung, wie in Anspruch 10 definiert, und mindestens einen pharmazeutisch akzeptablen Hilfsstoff.

13. Pharmazeutische Zusammensetzung nach Anspruch 12, wobei die pharmazeutische Zusammensetzung eine orale feste Darreichungsform ist.

14. Pharmazeutische Zusammensetzung nach Anspruch 13, wobei die orale feste Darreichungsform eine Tablette oder eine Kapsel ist.

15. Der in irgendeinem der Ansprüche 1 bis 9 definierte Co-Kristall, die in Anspruch 10 definierte Zusammensetzung oder die pharmazeutische Zusammensetzung nach Anspruch 12 bis 14 zur Verwendung bei der Behandlung und/oder Prophylaxe von rheumatoider Arthritis, atopischer Dermatitis, systemischem Lupus erythematosus (SLE), psoriatischer Arthritis und Psoriasis.

## Revendications

1. Co-cristal de baricitinib avec de l'acide lactique.

2. Co-cristal selon la revendication 1, dans lequel le rapport molaire du baricitinib sur l'acide lactique est de 1/1.

3. Co-cristal selon les revendications 1 ou 2, dans lequel l'acide lactique est de l'acide *L*-lactique.

4. Co-cristal selon les revendications ¹ ou 2, dans lequel l'acide lactique est de l'acide *D*-lactique.

5. Co-cristal selon l'une quelconque des revendications 1 à 3 **caractérisé en ce qu'**il présente la structure chimique selon la formule B1 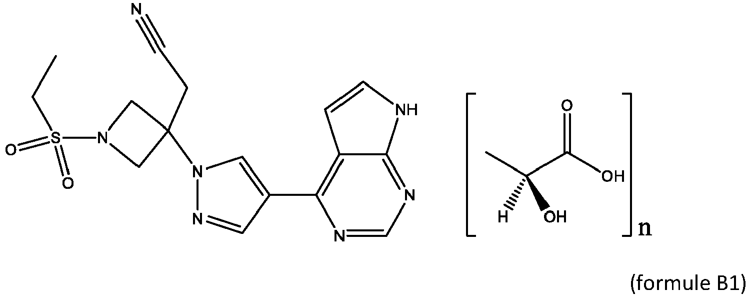 dans laquelle n se situe dans la plage allant de 0,8 à 1,2.

6. Co-cristal selon la revendication 1, 2 ou 4 **caractérisé en ce qu'**il présente la structure chimique selon la formule B2 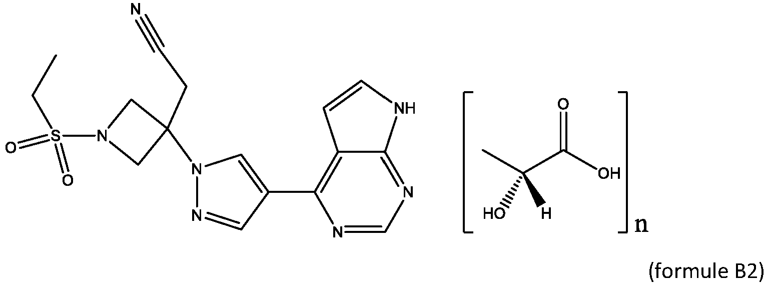 dans laquelle n se situe dans la plage allant de 0,8 à 1,2.

7. Co-cristal selon l'une quelconque des revendications précédentes **caractérisé en ce qu'**il présente un diffractogramme à rayons X sur poudre comprenant des réflexions à des angles 2-thêta de (7,2 ± 0,2)°, (9,0 ± 0,2)° et (10,7 ± 0,2)°, lorsqu'on le mesure à une température située dans la plage allant de 20 à 30 °C avec un rayonnement Cu-Kalpha_{1,2} présentant une longueur d'onde de 0,15419 nm.

8. Co-cristal selon l'une quelconque des revendications précédentes **caractérisé en ce qu'**il présente un spectre infrarouge par transformée de Fourier comprenant des pics à des nombres d'onde de (3482 ± 2) cm⁻¹, (1689 ± 2) cm⁻¹ et (1327 ± 2) cm⁻¹, lorsqu'on le mesure à une température située dans la plage allant de 20 à 30 °C avec une cellule de réflexion totale atténuée par diamant.

9. Co-cristal selon l'une quelconque des revendications précédentes **caractérisé en ce qu'**il présente un spectre Raman comprenant des pics à des nombres d'onde de (2931 ± 3) cm⁻¹, (1595 ± 3) cm⁻¹ et (1283 ± 3) cm⁻¹, lorsqu'on le mesure à une température située dans la plage allant de 20 à 30 °C et à une longueur d'onde de 785 nm.

10. Composition comprenant le co-cristal tel que défini selon l'une quelconque des revendications précédentes **caractérisée en ce qu'**elle présente un diffractogramme à rayons X sur poudre ne comprenant aucune réflexion à des angles 2-thêta de (16,32 ± 0,2)° et (20,40 ± 0,2)°, lorsqu'on la mesure à une température située dans la plage allant de 20 à 30 °C avec un rayonnement Cu-Kalpha_{1,2} présentant une longueur d'onde de 0,15419 nm.

11. Utilisation du co-cristal tel que défini selon l'une quelconque des revendications 1 à 9 ou de la composition telle que définie selon la revendication 10 pour la préparation d'une composition pharmaceutique.

12. Composition pharmaceutique comprenant le co-cristal tel que défini selon l'une quelconque des revendications 1 à 9 ou la composition telle que définie selon la revendication 10 et au moins un excipient pharmaceutiquement acceptable.

13. Composition pharmaceutique selon la revendication 12, dans laquelle la composition pharmaceutique est une forme pharmaceutique solide orale.

14. Composition pharmaceutique selon la revendication 13, dans laquelle la forme pharmaceutique solide orale est un comprimé ou une gélule.

15. Co-cristal tel que défini selon l'une quelconque des revendications 1 à 9, composition telle que définie selon la revendication 10 ou composition pharmaceutique selon la revendication 12 à 14 destiné(e) à être utilisé(e) dans le traitement et/ou en prophylaxie de la polyarthrite rhumatoïde, de la dermatite atopique, du lupus érythémateux disséminé (SLE), de l'arthrite psoriasique et du psoriasis.
